# EUROPEAN PATENT APPLICATION

(11) **EP 3 888 717 A1**
(43) Date of publication of application: **06.10.2021**
(21) Application number: 19891137.2
(22) Date of filing: 29.11.2019
(51) Int. Cl.: A61L 31/02, A61L 31/14, A61L 27/04, C22C 23/00, C22C 1/02

(54) **BIODEGRADABLE METAL ALLOY**

(30) Priority: 30.11.2018 KR 20180152118; 30.11.2018 KR 20190118726
(71) Applicant: U & I Corporation, Uijeongbu-si Gyeonggi-do 11781 (KR); Korea Institute of Science and Technology, Seoul 02792 (KR)
(72) Inventor: KOO, Ja Kyo, Namyangju-si, Gyeonggi-do 12111 (KR); JUNG, Hwa Chul, Seoul 08734 (KR); SHIM, Won Hyun, Uijeongbu-si, Gyeonggi-do 11772 (KR); ROH, Hyung Jin, Uijeongbu-si, Gyeonggi-do 11770 (KR); SEOK, Hyun Kwang, Seoul 01336 (KR); KIM, Yu Chan, Goyang-si, Gyeonggi-do 10532 (KR)
(74) Representative: Botti, Mario
(86) International application number: PCT/KR2019/016680
(87) International publication number: WO 2020/111854

(57) **Abstract**

Provided is a biodegradable metal alloy with multiple properties, containing: 0.05-0.15 wt% of calcium; a metal element X having a HCP structure, of a composition not forming a precipitated phase when mixed with magnesium; and magnesium as the remainder.

## Description

### [Technical Field]

The present disclosure relates to a biodegradable metal alloy, more particularly to a biodegradable metal alloy, which exhibits improved corrosiveness and strength as well as elongation and toughness by adding a dissimilar metal element to a magnesium alloy.

### [Background Art]

Recently, metals that are degraded in the body are being studied as implant and stent materials used for medical purposes.

The biodegradable metals should have superior mechanical properties to endure the torsional stress generated from a screw or the load generated from bone fracture when implanted in the body. Among them, toughness refers to the amount of energy capable of absorbing the torsional stress generated from the screw or the load generated from bone fracture, etc. In general, to improve the toughness, the improvement of yield point and elongation is necessary. For this, it is required to refine the metal alloy texture through additional processes such as quenching, sintering, heat treatment, etc. of the biodegradable metal and control the internal residual stress. In addition, the metal alloy used as a biodegradable metal should be designed appropriately in terms of added elements and alloy composition. The alloy composition is changed generally with the amount of elements added. The mechanical strength is improved as the amount of elements added to the alloy is increased.

However, the increase in the amount of the added elements leads to generation of metal-metal compounds or secondary phases, which causes the formation of a micro-galvanic circuit that accelerates corrosion. This increases the corrosion rate of the biodegradable metal. In addition, the elements added to the biodegradable metal may accelerate or decelerate the degradation of the biodegradable metal alloy by preventing or promoting galvanic corrosion. Accordingly, a biodegradable metal material which merely exhibits good mechanical properties and fast biodegradation rate cannot be applied to implants.

In this regard, Korean Patent Publication No. 10-2014-0099431 discloses a biodegradable implant containing magnesium, which contains, as impurities, manganese (Mn) and at least one selected from a group consisting of iron (Fe), nickel (Ni) and a mixture of iron (Fe) and nickel (Ni), wherein the content of the impurities is more than 0 and less than or equal to 1 part by weight based on 100 parts by weight of the magnesium and the ratio of {at least one selected from a group consisting of iron (Fe), nickel (Ni) and a mixture of iron (Fe) and nickel (Ni)} / manganese (Mn) is greater than 0 and smaller than or equal to 5, and a method for manufacturing the same.

However, the technology of ensuring satisfactory mechanical properties, particularly toughness, in consideration of elongation and strength while decreasing corrosion rate is still not enough.

### [Disclosure]

### [Technical Problem]

The present disclosure is directed to providing a biodegradable metal alloy based on a new composition, which has improved corrosiveness and mechanical properties, and a method for preparing the same.

### [Technical Solution]

The present disclosure provides a biodegradable metal alloy with multiple properties, which contains: 0.05-0.15 wt% of calcium; a metal element X having a HCP structure, of a composition not forming a precipitated phase when mixed with magnesium; and magnesium as the remainder.

In an exemplary embodiment of the present disclosure, the X is at least one selected from a group consisting of Sc, Gd, Dy, Y, Nd, Ho, Er, Tm, Lu and Zn.

In an exemplary embodiment of the present disclosure, the solid solubility of the X for magnesium is 5% or higher and the content of the X based on the total biodegradable metal alloy is 0.1 wt% or greater and less than 2 wt%.

In an exemplary embodiment of the present disclosure, the biodegradable metal alloy with multiple properties is extruded after casting and the biodegradable metal alloy with multiple properties has a toughness of 5000 or higher.

In an exemplary embodiment of the present disclosure, the biodegradable metal alloy with multiple properties has a corrosion rate of 0.005 mL/cm²/hr or lower in a phosphate-buffered saline (PBS) which simulates the in-vivo environment.

### [Advantageous Effects]

According to the present disclosure, by adding an element with a HCP structure, having high solid solubility for magnesium, and calcium together to magnesium, thereby improving mechanical properties (strength and elongation) and toughness, the resistance to torsional stress, load carrying capacity at the fracture site and corrosiveness can be improved at the same time when the metal alloy is used as a biodegradable implant. Accordingly, the biodegradable metal alloy may be used as a material for a biodegradable implant such as an implant for bone or ligament reinforcement, a stent, etc.

### [Best Mode]

The present disclosure provides a biodegradable metal alloy with multiple properties, which contains: 0.05-0.15 wt% of calcium; a metal element X having a HCP structure, of a composition not forming a precipitated phase when mixed with magnesium; and magnesium as the remainder.

The inventors of the present disclosure have developed an alloy with high toughness by adding magnesium, calcium and an element having the same hexagonal close-packing (Hereinafter, referred to as HCP) structure as magnesium, thereby improving strength and elongation.

In particular, the present disclosure can provide improved strength and elongation by adding an alloying element with a HCP structure, which has a high solid solubility for magnesium, and adding a trace amount of calcium element, thereby refining crystal grains.

But, when an element with a HPC structure, which has low solid solubility (lower than 5% for magnesium), is used, metal-metal compounds and precipitated phases may be formed easily. The precipitated phase acts as a cause of increasing corrosion rate in the in-vivo environment by forming a galvanic circuit. The added calcium element may improve mechanical properties by refining the crystal grain size. But, if it is added in an excessive amount, precipitated phases such as Mg₂Ca, etc. are formed excessively, resulting in increased corrosion rate. Accordingly, by designing an appropriate composition of an element having high solid solubility for magnesium and having a HCP structure (not forming a precipitated phase) and the Ca element, a biodegradable magnesium alloy with superior mechanical properties and corrosiveness may be provided.

A biodegradable metal alloy according to an exemplary embodiment of the present disclosure contains: less than 0.15 wt% of calcium based on magnesium; and a metal element X having a HCP structure, of a composition not forming a precipitated phase when mixed with magnesium. In the present disclosure, the X may be at least one selected from a group consisting of Sc, Gd, Dy, Y, Nd, Ho, Er, Tm, Lu and Zn, as an element having a solid solubility of 5 wt% or higher for magnesium.

In the present disclosure, the element having a HCP structure is used to decrease corrosion rate in vivo and to improve mechanical properties such as elongation, etc.

In an exemplary embodiment of the present disclosure, the content of the X is 0.1-1.5 wt% based on the total biodegradable metal alloy. If the content is below the lower limit, the actual effect of improving corrosiveness may be insignificant and superior mechanical properties may not be achieved. And, if it exceeds the upper limit, mechanical properties are improved but a large amount of hydrogen gas may be generated because of too high degradation rate.

Hereinafter, the present disclosure is described in more detail through specific examples. In embodiments which set forth below, a figure before each metal, excluding magnesium, represents the total wt% in magnesium alloy, and the remainder is magnesium. For instance, Mg-5Ca=1Zn represents metal alloy containing 5 wt% of calcium, 1 wt% of zinc and magnesium as the remainder.

### Examples

A magnesium ingot prepared using a stainless steel (SUS410) crucible was loaded in a crucible. The magnesium-loaded crucible was heated. When the temperature of the molten magnesium reached 700 °C or higher, zinc (Zn) and calcium (Ca) were loaded and the molten mixture of calcium, zinc and magnesium in the crucible was stirred to be mixed well with each other. The completely molten magnesium alloy was poured into a mold having a diameter of 50 mm and then water-cooled to refine the magnesium alloy texture.

Then, after surface-processing the cast magnesium alloy in solid state, direct extrusion was performed. In an example of the present disclosure, the extrusion speed was set to 0.1-0.3 mm/sec and the reduction in cross-sectional area before and after the extrusion (extrusion ratio) was set to 39:1.

The mechanical properties of biodegradable magnesium implant samples prepared as described above with the compositions described in Table 1 were evaluated according to ASTM-B557M-15. The result is summarized in Table 1.

**[Table 1]**

| | Mg alloy | UTS (MPa) | S.D (MPa) | Elongation (%) | S.D (%) | Yield strength (MPa) | Toughness |
|---|---|---|---|---|---|---|---|
| Comparative Example 1 | Pure Mg | 184.2 | 8.1 | 17.2 | 0.7 | 118.1 | 3250 |
| Comparative Example 2 | Mg-1Zn | 222.4 | 12.1 | 17.8 | 1.3 | 106.7 | 3773 |
| Comparative Example 3 | Mg-2Zn-0.1Ca | 242.7 | 2 | 25.3 | 1.4 | 148.6 | 5303 |
| Comparative Example 4 | Mg-3Zn-0.1Ca | 247.4 | 3.4 | 25.2 | 1.5 | 131.4 | 5232 |
| Comparative Example 5 | Mg-1Ca(FCC) | 229.2 | 7.9 | 13.3 | 4.7 | 186.8 | 1846 |
| Comparative Example 6 | Mg-5Ca-1Zn | 278.2 | 3.5 | 8.2 | 2.3 | 251.2 | 1656 |
| Example 1 | Mg-1Zn-0.1Ca | 230.3 | 4.1 | 30.1 | 2.9 | 206.4 | 7584 |
| Example 2 | Mg-1Sc-0.1Ca | 197.6 | 4.4 | 29.6 | 3.1 | 161.8 | 5667 |
| Example 3 | Mg-1Dy-0.1Ca | 193.4 | 3.2 | 30.3 | 1.9 | 148.8 | 5677 |
| Example 4 | Mg-1Gd-0.1Ca | 208.6 | 3.6 | 30.5 | 3.0 | 166.4 | 6019 |
| Example 5 | Mg-1Y-0.1Ca | 198 | 0.3 | 27.8 | 2.1 | 155.7 | 5640 |

Here, UTS represents Ultimate Tensile Strength and S.D represents Standard Deviation.

As shown in Table 1, the elongation was improved remarkably when an element with a HCP structure having a solid solubility for magnesium of 5 wt% or higher and 0.1 wt% of calcium were added. However, when zinc with a HCP structure was added in excess amounts (Comparative Examples 3 and 4), corrosiveness was increased rapidly as shown in Table 2. Accordingly, the adequate content of the HCP metal element in terms of the improvement of mechanical properties and in-vivo corrosiveness is 0.1-2 wt%.

Regarding toughness, as compared to the commercially available biodegradable magnesium alloy containing 5 wt% of calcium and 1 wt% of zinc (Comparative Example 6), Examples 1-5 within the composition ranges of the present disclosure showed about 4 times higher toughness (5000 or higher). This is due to the combination of the HCP element and Ca at the given composition ranges. In addition, the improvement in corrosiveness can be expected as shown in Table 2. Throughout the description, a figure before each metal represents wt% of the corresponding metal.

Table 2 shows a result of comparative analysis of in-vivo corrosiveness.

**[Table 2]**

| | Mg alloy | H2 gas generation rate (ml/cm²/hrs) | | | |
|---|---|---|---|---|---|
| | | Averg. (48hrs) | S.D | Averg. (168hrs) | S.D |
| Comparative Example 1 | Pure Mg | 0.010 | 0.001 | 0.006 | 0.001 |
| Comparative Example 3 | Mg-2Zn-0.1Ca | 0.014 | 0.001 | 0.009 | 0.003 |
| Comparative Example 4 | Mg-3Zn-0.1Ca | 0.023 | 0.007 | 0.031 | 0.014 |
| Comparative Example 5 | Mg-1Ca(FCC) | 0.062 | 0.011 | 0.133 | 0.017 |
| Comparative Example 6 | Mg-5Ca-1Zn | 0.016 | 0.005 | 0.010 | 0.020 |
| Example 1 | Mg-1Zn-0.1Ca | 0.005 | 0.001 | 0.004 | 0.001 |
| Example 2 | Mg-1Sc-0.1Ca | 0.010 | 0.001 | 0.005 | 0.001 |
| Example 3 | Mg-1Dy-0.1Ca | 0.010 | 0.001 | 0.005 | 0.000 |
| Example 4 | Mg-1Gd-0.1Ca | 0.009 | 0.001 | 0.004 | 0.001 |
| Example 5 | Mg-1Y-0.1Ca | 0.009 | 0.001 | 0.005 | 0.001 |

In Table 2, FCC represents Face Centered Cubic as a crystallographic terminology, which is different from HCP.

Referring to Table 2, it can be seen that the corrosion rate in a phosphate-buffered saline (PBS) which simulates the in-vivo environment decreases greatly when 0.15 wt% of calcium and 1 wt% of the HCP element X were added (hydrogen generation is proportional to the corrosion rate, 0.005 ml/cm2/hrs or lower).

When compared with the commercially available biodegradable magnesium alloy material (Comparative Example 6), those of the examples showed about 2 times superior corrosion resistance at 168 hours. In addition, when the content of X was increased to 2-3 wt% (Comparative Examples 3 and 4), the strength was improved but the corrosion rate was increased. It seems that the corrosion rate was increased because zinc or calcium was not completely dissolved and precipitated phases such as Mg₂Ca or Ca₂Mg₆Zn₃ were formed.

From the above results, it can be seen that an alloy with improved mechanical properties and good corrosion resistance can be prepared by adding less than 0.15 wt% of calcium and 0.1-1.5 wt% of the element X having a HCP structure (X = Sc, Gd, Dy, Y, Nd, Ho, Er, Tm, Lu or Zn) to Mg.

### [Industrial Applicability]

The present disclosure has industrial applicability in using metal alloy as orthopedic implants and the like.

## Claims

1. A biodegradable metal alloy with multiple properties, comprising:
0.05-0.15 wt% of calcium;
a metal element X having a HCP structure, of a composition not forming a precipitated phase when mixed with magnesium; and
magnesium as the remainder.

2. The biodegradable metal alloy with multiple properties according to claim 1, wherein the X is at least one selected from a group consisting of Sc, Gd, Dy, Y, Nd, Ho, Er, Tm, Lu and Zn.

3. The biodegradable metal alloy with multiple properties according to claim 2, wherein the solid solubility of the X for magnesium is 5% or higher.

4. The biodegradable metal alloy with multiple properties according to claim 3, wherein the content of the X based on the total biodegradable metal alloy is 0.1 wt% or greater and less than 2 wt%.

5. The biodegradable metal alloy with multiple properties according to claim 4, wherein the biodegradable metal alloy with multiple properties is extruded after casting and the biodegradable metal alloy with multiple properties has a toughness of 5000 or higher.

6. The biodegradable metal alloy with multiple properties according to claim 5, wherein the biodegradable metal alloy with multiple properties has a corrosion rate of 0.005 mL/cm²/hr or lower in a phosphate-buffered saline (PBS) which simulates the in-vivo environment.
